# EUROPEAN PATENT APPLICATION

(11) **EP 4 066 863 A1**
(43) Date of publication of application: **05.10.2022**
(21) Application number: 21397505.5
(22) Date of filing: 30.03.2021
(51) Int. Cl.: A61L 2/00, A61M 5/00, B65B 55/02, A61L 2/20, B01L 3/00, B01L 9/00

(54) **A PACKAGE, A METHOD AND USE**

(71) Applicant: Sartorius Biohit Liquid Handling Oy, 00880 Helsinki (FI)
(72) Inventor: KUITUNEN, Tuomas, 00880 Helsinki (FI); TIKKANEN, Tero, 00880 Helsinki (FI)
(74) Representative: Laine IP Oy

(57) **Abstract**

According to an example aspect of the present invention, there is provided a box-shaped package, comprising: a base, and a cover configured to be placed on the base to close the package, wherein in a closed configuration of the package, the interface between the base and the cover is gas-tight; the base and/or the cover comprise one or more gas-permeable zones which are capable of passing gases, such as air, into and out from the package, particularly in the closed configuration.

## Description

### FIELD

The present invention relates to packages for disposable laboratory consumables, particularly to packages for disposable pipette tips.

### BACKGROUND

Packages for disposable pipette tips are typically not hermetically gas-tight as such. Instead, purity of the contents is ensured by wrapping and encasing the package with a flexible plastic material, for example in the form of a bag or pouch.

One of the most often used wrapping methods is the flowpack process, in which a tightly sealed plastic bag is formed around the tip box. A disadvantage of the flowpack process is the air that remains inside the bag, which expands in high temperatures and in low pressures, which causes rupturing of the bag. Such problems are encountered particularly during sterilization and during high-altitude transport. A further disadvantage is that flowpack bags are difficult to open in a controlled manner, which increases contamination risk of the contents in an opening situation.

The problems related to expansion of the flexible bag during pressure and temperature variations can be avoided by using for example the shrinkwrap process in the manufacturing of the wrapping. In the shrinkwrap process, a thin plastic film is shrinked around the tip package by means of heat. The shrinkwrap film is full of tiny holes through which air escapes from inside the wrapping during the shrinking process. Shrinkwrap bags are easier to open than flowpack bags and they do not expand during pressure variations, since the film permeates air through the holes in it. However, a shrinkwrap bag does not provide gas-tightness or ensure purity of the contents.

It is known to place soft material between a base and a cover of a tip package in order to provide gas-tightness.

EP 1514602 A2 discloses a stand for pipette tips, comprising a base and a lid which provide a closed box. Between the base and the lid is a packing, which extends around a joint constituted thereby and which is made of a material softer than that used for the base and the lid.

There is a need for an improved package that ensures high purity of the contents.

There is a need for avoiding problems related to pressure and temperature variations during use, such as transport and sterilization of the package.

There is a need for a package that can be easily and reliably sterilized by means of a gas sterilization process.

It is an aim of the present invention to solve at least some of the problems in the known packages for disposable laboratory consumables.

### SUMMARY OF THE INVENTION

The invention is defined by the features of the independent claims. Some specific embodiments are defined in the dependent claims.

According to a first aspect of the present invention, there is provided a box-shaped package, comprising: a base, and a cover configured to be placed on the base to close the package, wherein in a closed configuration of the package, the interface between the base and the cover is gas-tight; the base and/or the cover comprise one or more gas-permeable zones which are capable of passing gases, such as air, into and out from the package, particularly in the closed configuration.

Various embodiments of the first aspect may comprise at least one feature from the following bulleted list:
- Said one or more zones are non-permeable to microbes.
- Said zones are configured to prevent build-up of a pressure difference between an interior of the package and the surroundings of the package in the closed configuration, for example during storage, transport and/or sterilization of the package.
- Said zones are configured to reduce pressure differences and humidity differences between an interior of the package and the surroundings of the package, particularly in the closed configuration.
- The material of said zones is more permeable to gases, such as oxygen and nitrogen, than the material of the other parts of the package, whereby the package mainly permeates gases through said zones in the closed configuration.
- The material of the zones comprises one or more selected from the following group: a paper-based material, Tyvek.
- In the closed configuration said one or more zones are separate from the interface between the base and the cover.
- Outside said one or more zones the base and the cover are not capable of passing gases into or out from the package.
- The material of the base and of the cover comprises or consists of a thermoplastic material, such as polypropylene.
- The package comprises: a box-shaped base comprising four lateral sidewalls and a bottom face, the base having an open top end in an opened configuration; a box-shaped cover comprising four lateral sidewalls and a top face, the cover having an open bottom end in an opened configuration; a plate-formed tip tray comprising openings adapted for receiving and holding disposable tips for a liquid handling device, said tip tray being located in an interior of the base; a first seal element on a perimeter of the open top end of the base and/or a second seal element on a perimeter of the open bottom end of the cover; wherein, in a closed configuration of the package, said cover lies on the base, the open ends of the base and the cover face each other, and the perimeters of the base and the cover engage with each other via said first seal element and/or second seal element; wherein said zones are located in one or more of the following: the lateral sidewalls of the base, the bottom face of the base, the lateral sidewalls of the cover, and the top face of the cover.
- At least one zone is located in the bottom face of the base.
- Said first and/or second seal elements are configured to hermetically seal the interface between the base and the cover in the closed configuration of the package.
- Said seal elements run on and along the entire perimeter of the open top end of the base and/or on and along the entire perimeter of the open bottom end of the cover.
- Said seal elements are integral to the base and/or the cover, or said seal elements constitute a separate detachable part or parts.
- The material of the seal elements comprises or consists of thermoplastic elastomer material, preferably selected from the following group: thermoplastic polyolefin elastomers, thermoplastic polyurethanes, thermoplastic copolyesters, thermoplastic polyamides, thermoplastic vulcanizates, styrenic block copolymers and combinations thereof.
- The package is hermetically re-sealable such that said first and/or second seal elements are configured to repeatedly hermetically seal the interface between the base and the cover upon repeated closures of the package.
- Said package contains disposable tips for a liquid handling device, such as a pipette, wherein said tips are supported by a plate-formed tip tray located in an interior of the base, and wherein said tips have been inserted into respective openings in the tip tray.

According to a second aspect of the present invention, there is provided a method, comprising: providing the package according to the first aspect, preferably containing plastic disposable tips for a liquid handling device, sterilizing said package, for example by a gas sterilization process or a radiation sterilization process.

Various embodiments of the second aspect may comprise at least one feature from the following bulleted list:
- Sterilizing is carried out by a gas sterilization process in which a bactericidal gas is used as a sterilizing gas.
- Said zones are permeable to said bactericidal gas.
- Sterilizing is carried out by a gas sterilization process in which ethylene oxide is used as a sterilizing gas.
- Said zones are permeable to ethylene oxide.
- Said zones are non-permeable to microbes that the bactericidal gas is capable of exterminating during said sterilization process.

According to a third aspect of the present invention, there is provided use of gas sterilization or radiation sterilization for sterilizing the package according to the first aspect.

The present invention provides many advantages.

Some embodiments of the present invention ensure a high level of purity for the contents of the package.

Some embodiments of the present invention provide a package that can be effectively gas-sterilized so that the sterilizing gas is able to access the interior of the package and to come into contact with the contents of the package.

Some embodiments of the present invention provide a package that is breathable (gas-permeable) and simultaneously non-permeable to air-born contaminants, such as microbes.

The present invention may reduce contamination of the contents also during storage of the package, because the contamination barrier property is provided by the package itself and not merely by a plastic bag encasing the package. In some embodiments of the present invention, a package that has been taken out of a plastic bag, opened, and partially consumed can again be rendered hermetically sealed by closing the cover of the package.

The present invention avoids problems that might arise if a hermetically sealed package is being opened in a temperature that is lower than manufacturing temperature or in a higher ambient pressure than the ambient pressure during manufacturing: In a completely gas-tight package, the underpressure inside the package would complicate opening of the package. However, in the present invention, the package is breathable, which evens out such pressure differences and facilitates opening.

### BRIEF DESCRIPTION OF THE DRAWINGS

FIGURES 1A and 1B illustrate as an exploded view a package in accordance with one embodiment of the present invention;
FIGURES 2A and 2B illustrate as an exploded view a package in accordance with another embodiment of the present invention;
FIGURE 3 illustrates the package of FIG. 1B as a three-dimensional view, in an opened configuration;
FIGURES 4A and 4B illustrate the package of FIG. 1B as three-dimensional views, in a closed configuration; and
FIGURES 5A to 5D illustrate the package of FIG. 1B as two-dimensional projections.

### EMBODIMENTS

### DEFINITIONS

In the present context, the term "microbial barrier" refers to a material or layer that provides a barrier against movement of microbes through the material or layer.

In the present context, the term "hermetically sealed" refers to a gas tight sealing or closure.

In the present context, the term "gas sterilization" refers to sterilization by means of a bactericidal gas. Gas sterilization is typically used for items that are heat and moisture sensitive. Ethylene oxide is the gas most often used; it may be diluted with carbon dioxide or fluorinated hydrocarbons. Gas sterilization involves a chemical reaction between chemical groups in the bacterial cell and the gas. Factors influencing gas sterilization include at least time of exposure, gas concentration, penetration of the gas, and temperature and humidity in the sterilizing chamber. Automatically controlled ethylene oxide sterilizers are usually heated to a temperature of about or at least 54 °C. A humidity level of 35 to 70 per cent is typically used.

In the present context, the term "radiation sterilization" refers to sterilization by application of radiation to an object to be sterilized.

The present invention provides an improved package for disposable laboratory consumables requiring a high purity level and sterilizability.

It has been surprisingly discovered that by providing the package with controlled permeability, many advantages may be achieved during typical uses of the package. Particularly, incorporation of gas permeable zones to the package and increase of gas-tightness of the cover-base interface together provide a package which can be efficiently sterilized and which can be hermetically re-closed in the course of laboratory work.

In some embodiments, the present invention provides a box-shaped package, comprising: a base, and a cover configured to be placed on the base to close the package, wherein: in a closed configuration of the package, the interface between the base and the cover is gas-tight; the base and/or the cover comprise one or more gas-permeable zones which comprise material that is permeable to gases, wherein in the closed configuration said gas-permeable zones are capable of passing gases, such as air, into the package.

Preferably, said one or more gas-permeable zones are non-permeable to microbes.

Preferably, the base and/or the cover comprise one or more gas-permeable zones which comprise material that is permeable to gases and non-permeable to microbes.

In one embodiment, said gas-permeable zones are configured to prevent build-up of a pressure difference between an interior of the package and the surroundings of the package in the closed configuration, for example during storage, transport and/or sterilization of the package.

In one embodiment, said zones are configured to reduce pressure differences and humidity differences between an interior of the package and the surroundings of the package, particularly in the closed configuration. Typically, evening of eventual pressure and/or humidity differences occurs mainly or exclusively via said gas permeable zones in the closed configuration.

The gas permeable zone is preferably permeable to water vapour, typically more permeable to water vapour than what polypropylene is.

In one embodiment, said gas-permeable zones are more permeable to gases, such as oxygen and nitrogen, than the material of the other parts of the package, whereby the package mainly or exclusively permeates gases through said gas-permeable zones in the closed configuration.

The gas permeable zone is preferably permeable to oxygen and nitrogen, typically more permeable to oxygen and nitrogen than what polypropylene is.

In one embodiment, in the closed configuration said one or more gas-permeable zones are separate from the interface between the base and the cover. Typically said one or more gas-permeable zones are located in the package structure at a distance from said interface and the seal element(s) in order to not interfere with hermetic sealing of said interface.

In some embodiments, said gas-permeable zone comprises a barrier element that is configured to selectively permeate certain gases and to stop passage of contaminants.

Permeability to gases through said gas-permeable zones preferably makes the package breathable. Passing of gases through the gas-permeable zones may reduce harmful effects due to ambient pressure variations, such as uncontrolled gas leakage through the interface between the cover and the base, and build-up of a pressure difference between the inner space of the package and the surroundings.

Passing of gases through the gas-permeable zones may enable effective gas sterilization of the contents, as the sterilizing gases are able to enter the interior of the package and to contact the articles to be sterilized, such as pipette tips.

In some embodiments, the gas-permeable zones comprise a breathable material, such as a porous paper-based material.

In some embodiments, the gas-permeable zones comprise one or more of the following materials: uncoated or coated Tyvek, uncoated or coated medical-grade paper.

In one embodiment, the one or more gas-permeable zones are constructed as a filter made of Tyvek.

Tyvek is an advantageous material as it provides a sufficient barrier to prevent contamination of the items in the package while still preventing the package from swelling, as Tyvek is a breathable material. Tyvek comprises HDPE (high-density polyethylene) fibres. Its porous fiber structure enables gases and vapours that are used in sterilization to pass through the material while the material still provides a good microbial barrier.

In another embodiment, the gas-permeable zones are made of medical-grade paper, preferably sterile-grade medical-grade paper. Preferably the medical-grade paper has a grammage of at least 35 lb, such as at least 70 lb, for example 35 to 100 lb, expressed as basis weight.

In one embodiment, the grammage of the material of the gas-permeable zones is in the range 30 to 170 g/m², in one example less than 120 g/m², for example less than 100 g/m².

The one or more gas-permeable zones may be attached to the material of the package by an in-mould labelling (IML) process during manufacturing of the package by injection moulding. Alternatively, the gas-permeable zones may be attached in a separate welding step after an injection moulding step of the other parts of the package.

In some embodiments, the gas-permeable zones are more permeable to atmospheric gases and moisture than the material of the base and the material of the cover.

In some embodiments, the gas-permeable zones are permeable to at least the following atmospheric gases: nitrogen, oxygen, carbon dioxide, water vapour.

In some embodiments, the gas-permeable zones function as a microbial barrier.

Microbial barrier performance can be measured by means of the following standards: ASTM F1608 and ASTM F2638, preferably the latter one.

In one embodiment, at least one gas-permeable zone is located in the base and/or in the cover. Preferably at least one gas permeable zone is located in the bottom face of the base, because in this way the functioning and structure of the zone during storage or transport may become less disturbed and be less vulnerable to damage.

The total area of the gas-permeable zones is preferably less than 50%, such as less than 10% of the total area of the outer surfaces (i.e. the lateral sidewalls and the top and bottom faces of the cover and the base) of the package. In this way sufficient rigidity of the package structure may be ensured.

The total area of the gas-permeable zones is preferably at least 0.1%, such as at least 1%, for example at least 5% of the total area of the outer surfaces (i.e. the lateral sidewalls and the top and bottom faces of the cover and the base) of the package. In this way efficient evening of pressure and/or humidity differences between the interior of the package and the surroundings may be ensured.

In an exemplary embodiment, the package comprises a box-shaped base comprising four lateral sidewalls and a bottom face, the base having an open end (open top) that is adapted to be covered by a cover. The cover is also box-shaped and comprises four lateral sidewalls and a top face, the cover also having an open end (open bottom). In a closed configuration of the package, the open ends of the base and the cover are arranged to face each other.

The package is typically intended for storing disposable tips for a liquid handling device. In such embodiments the package comprises a plate-formed tip tray comprising openings adapted for receiving and holding disposable tips. The tip tray is parallel to the bottom face of the base. The openings are typically in a matrix layout. The tip tray is fixed to the base and, together with the tips therein, becomes encased by the base and the cover in the closed configuration. The tip tray may for example be fixed to or supported by inner surfaces of the base.

Preferably, the package comprises a first seal element on a perimeter of the open end of the base and/or a second seal element on a perimeter of the open end of the cover. Said seal elements form a hermetic sealing between the base and the cover in a closed configuration of the package. Therefore, the seal element or the seal elements together run along and occupy the entire interface and contact area between the base and the cover.

Thus, in a closed configuration of the package, the cover lies on the base, the open ends of the base and the cover face each other, and the perimeters of the base and the cover engage with each other via said first seal element and/or second seal element.

The gas-permeable zones may be located in one or more of the following: the lateral sidewalls of the base, the bottom face of the base, the lateral sidewalls of the cover, and the top face of the cover.

In some embodiments of the present invention, the package comprises a gas-tight or hermetic seal between the cover and the base of the package. The package is closed by placing the cover on the base. The gas-tight seal is located in the interface (on the contact surfaces) between the cover and the base and provides a gas-tight barrier. Preferably the gas-tight seal prevents, at the interface, substantially all gas exchange between the interior space of the package and the surroundings, particularly exchange of gases in ambient air and gases used in sterilizing processes.

The material of the seal element(s) comprises or consists of thermoplastic elastomer material, preferably selected from the following group: thermoplastic polyolefin elastomers, thermoplastic polyurethanes, thermoplastic copolyesters, thermoplastic polyamides, thermoplastic vulcanizates, styrenic block copolymers and combinations thereof.

In one embodiment of the present invention, leakage of gases through the interface between the cover and the base of the package is completely prevented. The means for preventing leakage is preferably a sealing made of an elastic material.

Typically, the package comprises a base, a cover, a hinge connecting the cover to the base, and a seal element that is configured to be located between the facing surfaces of the cover and the base to provide a gas-tight seal to the entire interface between the cover and the base when the package is closed so that the package is hermetically sealed at the cover-base interface in the closed configuration.

The seal element may be attached and integral to the base or alternatively to the cover. In a further embodiment, both the base and the cover comprise a seal element, and the two seal elements are configured to contact and face each other in a closed configuration of the package.

Gas-tight closure is created upon placing the cover on the base. Preferably, a hinge is provided to connect the cover and the base and to direct the cover in a proper orientation onto the base upon closing the package.

Preferably, the structure of the hinge is designed to ensure reliable functioning of the seal, particularly a reliable contact of the seal with the entire target surface, which typically is a perimeter of the cover or the perimeter of the base or a second seal attached to such perimeter.

Additionally, the package may comprise a lock mechanism to lock the cover to the base in the closed configuration.

Preferably, the base and the cover are made of a plastic material, such as polypropylene.

In one embodiment, the base and the cover are manufactured by injection moulding.

The present invention also provides a method, comprising: providing the package, preferably containing plastic disposable tips for a liquid handling device, and sterilizing said package, for example by a gas sterilization process or a radiation sterilization process.

In some embodiments, sterilizing is carried out by a gas sterilization process in which a bactericidal gas is used as a sterilizing gas, and wherein said zones are permeable to said bactericidal gas. The bactericidal gas is able to pass through the material of the gas-permeable zones in order to sterilize the contents of the package. Due to the presence of gas-permeable zones, no pressure difference is created between the interior and the surroundings of the package. Sterilizing can be performed when the package is in a closed configuration and the interface between the base and the cover is hermetically sealed.

In some embodiments, sterilizing is carried out by a gas sterilization process in which ethylene oxide is used as a sterilizing gas, and wherein said zones are permeable to ethylene oxide.

In some embodiments, said zones are non-permeable to microbes which the bactericidal gas is capable of exterminating during said sterilization process.

The present invention also provides use of gas sterilization or radiation sterilization for sterilizing the package.

The present breathable package may be advantageous in a gas sterilization process, such as ethylene oxide sterilization, chlorine dioxide sterilization, or steam sterilization, and/or in a radiation sterilization process. In such sterilization processes it is expedient that eventual pressure and temperature variations during the process can be evened out and that sterilizing gases have unhindered access to the interior of the package.

During gas sterilization, the sterilizing gas must be able to enter the interior of the package in order to exterminate microbes inside the package and on the surfaces of the contents, such as pipette tips. By means of the present invention, sterilizing gases may permeate said gas-permeable zones and freely enter the package. Preferably, the gas-permeable zones are permeable to at least the sterilizing gas ethylene oxide.

It is advantageous that the package comprises at least two gas-permeable zones, preferably on different sides or faces of the package, so that diffusion of sterilizing gases into the package takes place more effectively and the sterilization process can be carried out more quickly. In one embodiment, the package comprises 2 to 6 gas-permeable zones, such as 2 to 4 gas-permeable zones. By having multiple gas-permeable zones located on separate faces of the package it is possible to ensure that gas exchange is not stopped in case that one of the faces is not properly exposed to the surrounding gaseous atmosphere or in case that gas permeability of one of the zones becomes reduced or hindered for some reason.

During radiation sterilization, the radiation imposed on the package raises the temperature inside the package, whereby also pressure increases. High pressure may damage the package and cause deformations and leakages, which may lead to an outflow of air from inside the package and later, when the radiation has been stopped and the temperature decreases, to an inflow of contaminated air into the package. Similar problems may arise when the package is transported to high altitudes and then brought back to lower altitudes. By means of the present invention, such pressure differences and consequent deformations and leakages may be prevented.

In preferred embodiments, the base, the cover and the hinge are configured to provide an even sealing pressure on the seal element between the base and the cover in the closed configuration of the package. Such even sealing pressure may be achieved for example by providing flexibility to the joint point of the hinge, to compensate for example for eventual dimensional variations in the structure of the package. Such dimensional variation may arise for example as a result of injection moulding of the package and also during temperature variations.

In one embodiment, the base, the cover and the seal between them are configured to provide an even sealing pressure at the interface despite dimensional variations up to ± 1 mm in the dimensions of the base and the cover.

In some embodiments, the package may be wrapped with a plastic film material, such as shrinkwrap film, which functions as a dust barrier.

Turning now to the drawings :

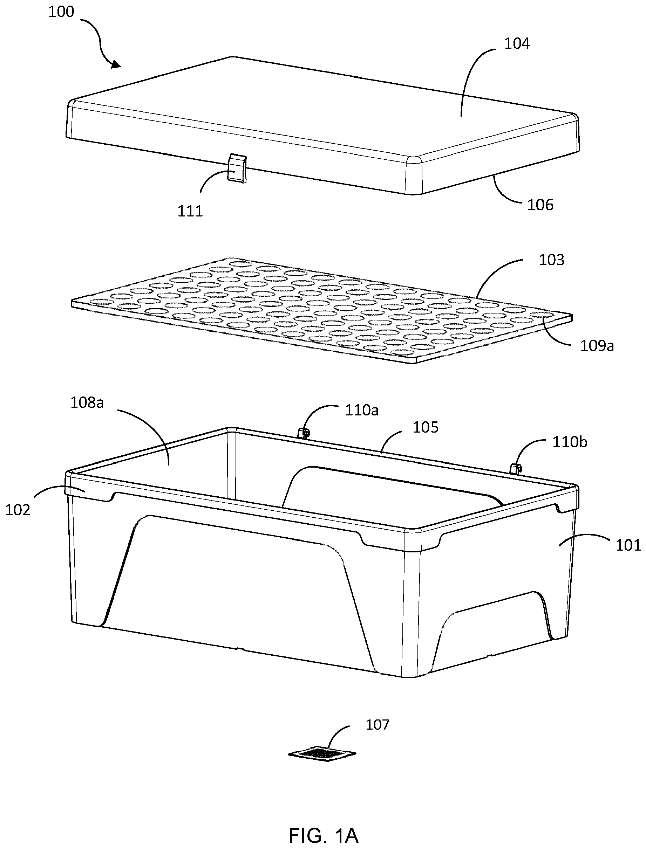

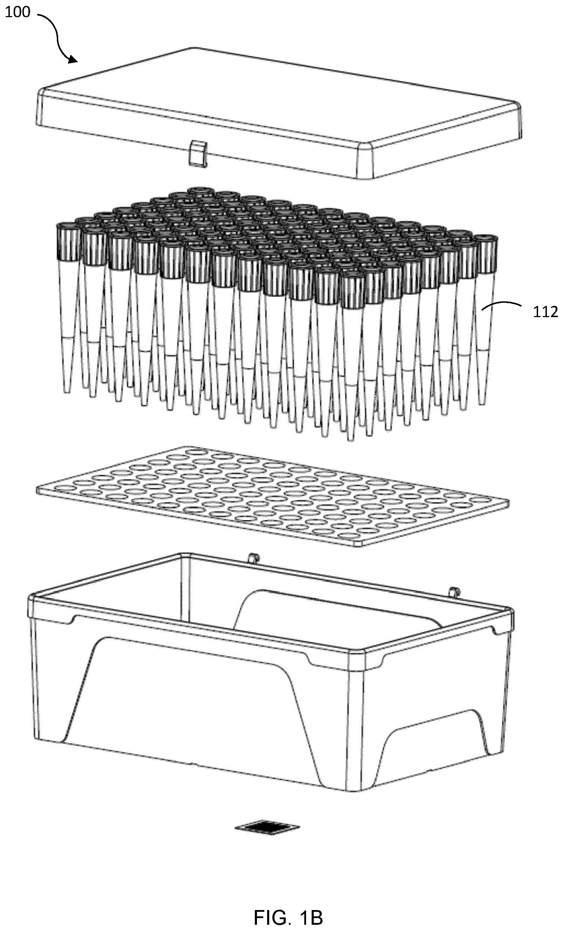

FIGURES 1A and 1B illustrates as an exploded view a package in accordance with at least some embodiments of the present invention.

As shown in FIG. 1A, the package 100 comprises a base 101, a seal element 102 integrated to the base 101, a tip tray 103, and a cover 104. The seal element 102 is fixed to and runs on the perimeter 105 (not visible) of the top end of the base along the entire length of the perimeter. In the closed configuration the perimeters 105, 106 of the base and the cover engage with each other via the seal element, and the seal element thus forms a hermetically sealed interface between the base and the cover. The package further comprises a square-shaped gas-permeable zone 107 as integrated to the bottom face of the base. The tip tray 103 is supported by the base, typically by suitable shapes (not shown) in the inner surfaces, such as the inner surface 108a, of the lateral sidewalls of the base, and becomes located in the interior of the package in the closed configuration. The tip tray comprises openings, such as the opening 109a, in a matrix layout, which openings are configured to receive and support disposable tips, such as plastic pipette tips. The package further comprises hinges 110a, 110b and a lock 111.

FIG. 1B shows the package 100 of FIG. 1A and additionally disposable pipette tips 112 to be contained in the package and supported by the tip tray.

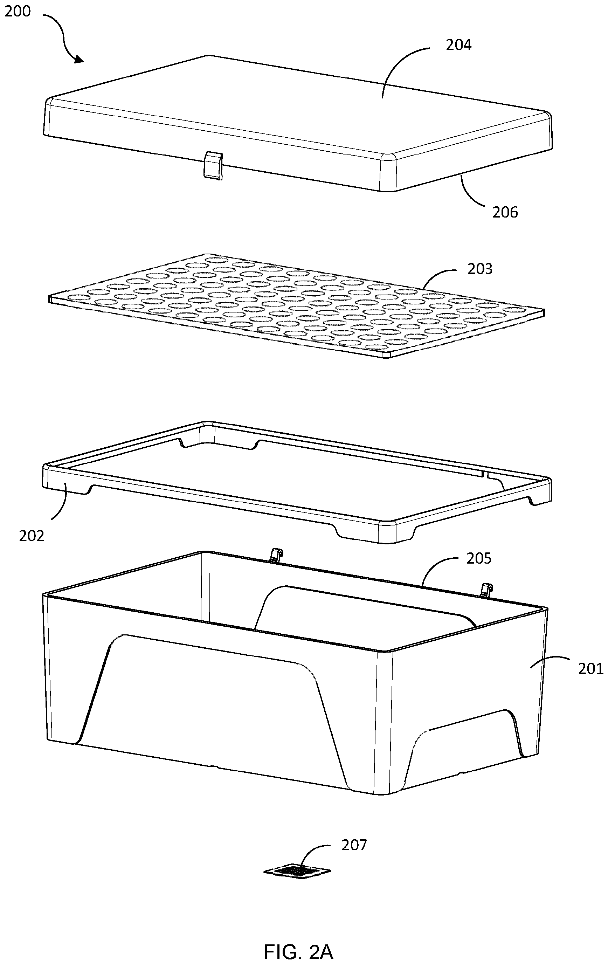

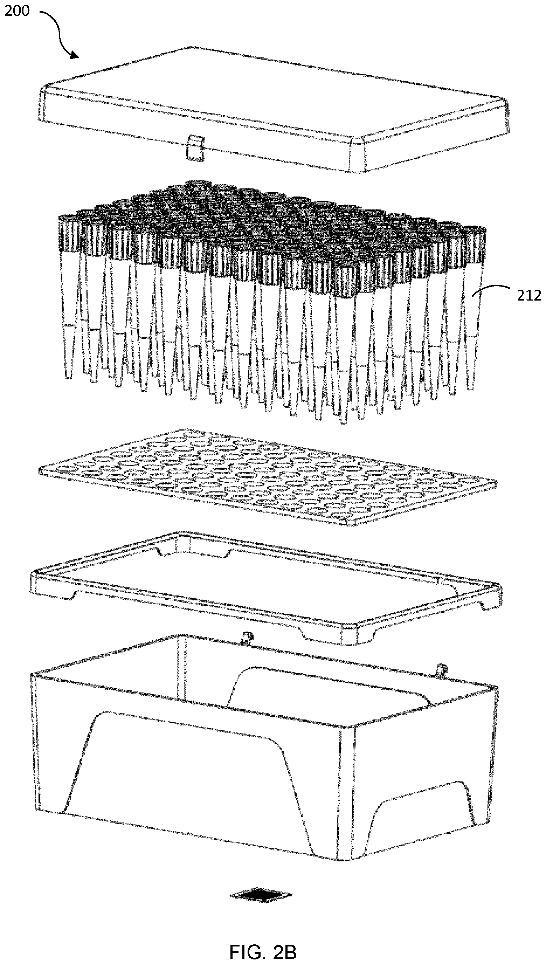

In the embodiment shown in FIGS. 2A and 2B the package 200 comprises a separate seal element 202, which in the closed configuration is located between the base 201 and the cover 204 in a corresponding way as the seal element 102 in FIGS. 1A and 1B but as a discrete element that is not fixed to either of the perimeters 205, 206 of the base 201 and the cover 204, respectively. The seal element 202 forms a hermetically sealed interface between the base and the cover as they face each other in the closed configuration.

FIG. 2B shows the package 200 of FIG. 2A and additionally disposable pipette tips 212 to be contained in the package and supported by the tip tray 203.

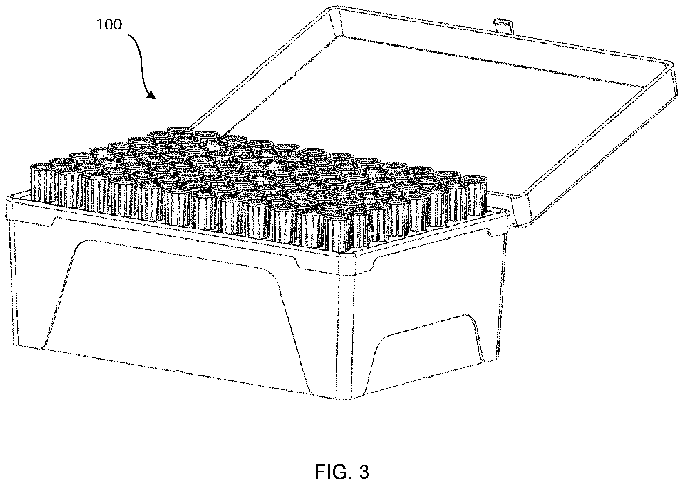

FIGURE 3 illustrates the package 100 of FIG. 1B as a three-dimensional view, in an opened configuration and with the pipette tips held by the tip tray.

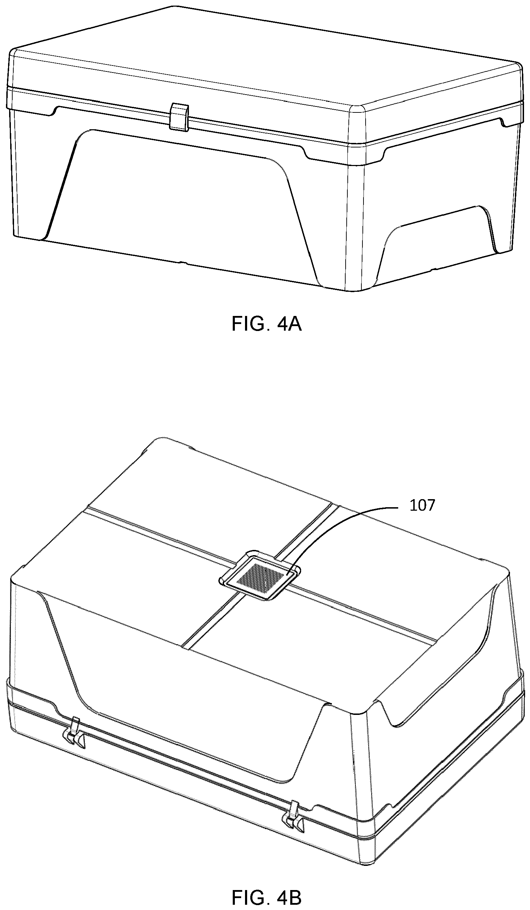

FIGURES 4A and 4B illustrate the package 100 of FIG. 1B as three-dimensional views, in a closed configuration. FIG. 4B shows the gas-permeable zone 107 in the bottom face of the base.

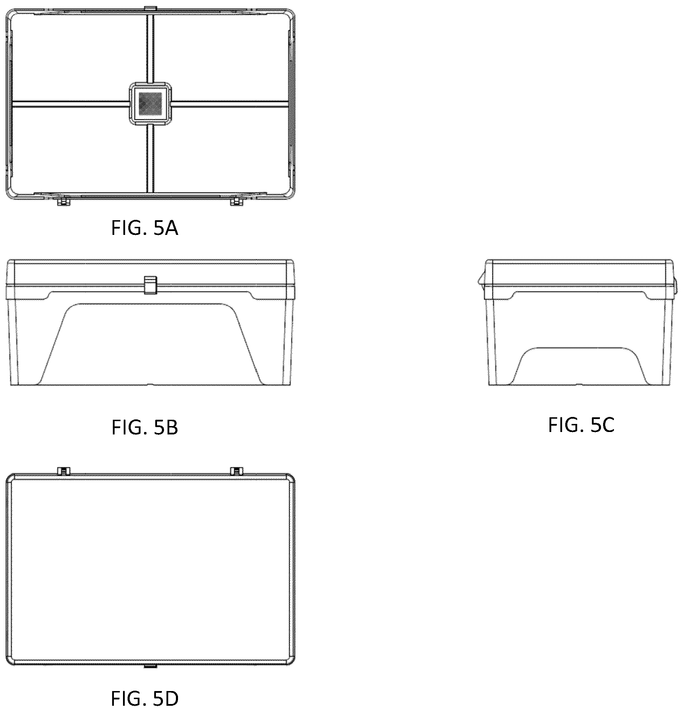

FIGURES 5A to 5D illustrate the package 100 of FIG. 1B as two-dimensional projections from below (FIG. 5A), from sides (FIGS. 5B and 5C), and from above (FIG. 5D), all in the closed configuration.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In the following description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", i.e. a singular form, throughout this document does not exclude a plurality.

### INDUSTRIAL APPLICABILITY

The present invention is applicable at least in packaging items that require a controlled purity level, such as pipette tips.

### ACRONYMS LIST

- IML: in-mould labelling
- HDPE: high-density polyethylene

### REFERENCE SIGNS LIST

- 100,200: package
- 101: base
- 102, 202: seal element
- 103, 203: tip tray
- 104: cover
- 105, 205: perimeter of the base
- 106, 206: perimeter of the cover
- 107, 207: gas-permeable zone
- 108a: inner surface of a lateral sidewall of the base
- 109a: opening
- 110a, 110b: hinges
- 111: lock
- 112,212: pipette tips

### CITATION LIST

### Patent Literature

EP 1514602 A2

## Claims

1. A box-shaped package, comprising:
- a base, and
- a cover configured to be placed on the base to close the package,
**characterized in that**:
- in a closed configuration of the package, the interface between the base and the cover is gas-tight;
- the base and/or the cover comprise one or more gas-permeable zones which are capable of passing gases, such as air, into and out from the package, particularly in the closed configuration.

2. The package according to claim 1, wherein said one or more gas-permeable zones are non-permeable to microbes.

3. The package according to any of the preceding claims, wherein said one or more gas-permeable zones are configured to prevent build-up of a pressure difference between an interior of the package and the surroundings of the package in the closed configuration, for example during storage, transport and/or sterilization of the package.

4. The package according to any of the preceding claims, wherein said one or more gas-permeable zones are configured to reduce pressure differences and humidity differences between an interior of the package and the surroundings of the package, particularly in the closed configuration.

5. The package according to any of the preceding claims, wherein the material of said one or more gas-permeable zones is more permeable to gases, such as oxygen and nitrogen, than the material of the other parts of the package, whereby the package mainly permeates gases through said one or more gas-permeable zones in the closed configuration.

6. The package according to any of the preceding claims, wherein the material of said one or more gas-permeable zones comprises one or more selected from the following group: a paper-based material, Tyvek.

7. The package according to any of the preceding claims, wherein in the closed configuration said one or more gas-permeable zones are separate from the interface between the base and the cover.

8. The package according to any of the preceding claims, wherein outside said one or more gas-permeable zones the base and the cover are not capable of passing gases into or out from the package.

9. The package according to any of the preceding claims, wherein the material of the base and of the cover comprises or consists of a thermoplastic material, such as polypropylene.

10. The package according to any of the preceding claims, comprising:
- a box-shaped base comprising four lateral sidewalls and a bottom face, the base having an open top end in an opened configuration;
- a box-shaped cover comprising four lateral sidewalls and a top face, the cover having an open bottom end in an opened configuration;
- a plate-formed tip tray comprising openings adapted for receiving and holding disposable tips for a liquid handling device, said tip tray being located in an interior of the base;
- a first seal element on a perimeter of the open top end of the base and/or a second seal element on a perimeter of the open bottom end of the cover;
- wherein, in a closed configuration of the package, said cover lies on the base, the open ends of the base and the cover face each other, and the perimeters of the base and the cover engage with each other via said first seal element and/or second seal element;
- wherein said one or more gas-permeable zones are located in one or more of the following: the lateral sidewalls of the base, the bottom face of the base, the lateral sidewalls of the cover, and the top face of the cover.

11. The package according to any of the preceding claims, wherein at least one zone of said one or more gas-permeable zones is located in the bottom face of the base.

12. The package according to any of the preceding claims, wherein said first and/or second seal elements are configured to hermetically seal the interface between the base and the cover in the closed configuration of the package.

13. The package according to any of the preceding claims, wherein said seal elements run on and along the entire perimeter of the open top end of the base and/or on and along the entire perimeter of the open bottom end of the cover.

14. The package according to any of the preceding claims, wherein said seal elements are integral to the base and/or the cover, or said seal elements constitute a separate detachable part or parts.

15. The package according to any of the preceding claims, wherein the material of the seal elements comprises or consists of thermoplastic elastomer material, preferably selected from the following group: thermoplastic polyolefin elastomers, thermoplastic polyurethanes, thermoplastic copolyesters, thermoplastic polyamides, thermoplastic vulcanizates, styrenic block copolymers and combinations thereof.

16. The package according to any of the preceding claims, wherein the package is hermetically re-sealable such that said first and/or second seal elements are configured to repeatedly hermetically seal the interface between the base and the cover upon repeated closures of the package.

17. The package according to any of the preceding claims, wherein said package contains disposable tips for a liquid handling device, such as a pipette, wherein said tips are supported by a plate-formed tip tray located in an interior of the base, and wherein said tips have been inserted into respective openings in the tip tray.

18. A method, comprising:
- providing the package according to any of the preceding claims, preferably containing plastic disposable tips for a liquid handling device,
- sterilizing said package, for example by a gas sterilization process or a radiation sterilization process.

19. The method according to claim 18, wherein sterilizing is carried out by a gas sterilization process in which a bactericidal gas is used as a sterilizing gas, and wherein said one or more gas-permeable zones are permeable to said bactericidal gas.

20. The method according to claim 18 or claim 19, wherein sterilizing is carried out by a gas sterilization process in which ethylene oxide is used as a sterilizing gas, and wherein said one or more gas-permeable zones are permeable to ethylene oxide.

21. The method according to any of claim 18 to 20, wherein said one or more gas-permeable zones are non-permeable to microbes that the bactericidal gas is capable of exterminating during said sterilization process.

22. Use of gas sterilization or radiation sterilization for sterilizing the package according to any of claims 1 to 17.
